# EUROPEAN PATENT SPECIFICATION

(11) **EP 1 480 616 B1**
(45) Date of publication and mention of the grant of the patent: **17.11.2010**
(21) Application number: 03743339.8
(22) Date of filing: 26.02.2003
(51) Int. Cl.: A61K 9/00, A61K 31/485

(54) **Aerosol formulations of diisobutyryl apomorphine**
Aerosol-formulierungen von diisobutyryl apomorphine
Formulations aerosol de diisobutyryl apomorphine

(30) Priority: 01.03.2002 EP 02004786
(43) Date of publication of application: 01.12.2004
(73) Proprietor: CHIESI FARMACEUTICI S.p.A., 43100 Parma (IT)
(72) Inventor: DAVIES, Rebecca, Jaine, 43100 Parma (IT); GANDERTON, David, I-43100 Parma (IT); LEWIS, David, Andrew, I-43100 Parma (IT); MEAKIN, Brian, John, I-43100 Parma (IT); BRAMBILLA, Gaetano, I-43100 Parma (IT); FERRARIS, Alessandra, I-43100 Parma (IT)
(74) Representative: Minoja, Fabrizio
(86) International application number: PCT/EP2003/001962
(87) International publication number: WO 2003/074023

(56) References cited:
- WO-A-00/30608
- WO-A-01/49350
- WO-A-01/62227
- WO-A-01/66089
- WO-A-01/74358
- WO-A-98/24420
- GB-A- 2 326 334
- GB-A- 2 354 007

## Description

### Field of the invention

This invention relates to aerosol solution formulations as claimed in claim 1.

### Background of the invention

Many pharmaceutically active compounds currently used in clinical practice and exhibiting problems of administration and/or absorption by the oral, parenteral or transdermal administration could take advantage from a pulmonary delivery, aimed at obtaining a systemic effect.

Pharmaceutically active compounds could be administered to the respiratory tract by using pressurised metered dose inhalers (pMDIs). PMDIs use a propellant to expel droplets containing the pharmaceutical product to the respiratory tract as an aerosol.

The formulation can be a solution or a suspension. Solution formulations, in comparison to suspensions, do not present problems of physical stability of the suspended particles and could therefore guarantee a higher dose uniformity and reproducibility.

As far as the propellant is concerned, hydrofluoroalkanes [(HFAs) known also as hydro-fluoro-carbons (HFCs)] would be mandatory propellants as chlorofluorocarbons (known also as Freons or CFCs), which were for many years the preferred aerosol propellants for pharmaceutical use, have been banned in view of their environmental impact.

In particular, 1,1,1,2-tetrafluoroethane (HFA 134a) and 1,1,1,2,3,3,3-heptafluoropropane (HFA 227) have been acknowledged to be the best candidates for non-CFC propellants and a number of pharmaceutical aerosol formulations using such HFA propellants have been disclosed.

GB 2 326 334 discloses a composition for use in an aerosol inhaler, comprising an active material, a HFA propellant, a cosolvent and a low colatility component to increase the mass median aerodynamic diameter (MMAD) of the aerosol particles.

GB 2 354 007 discloses a formulation comprising fluticasone propionate in a HFA propellant, and a low volatility component to increase the MMAD of the aerosol particles on actuation of the inhaler.

WO 98 24 420 refers to a device for providing pharmaceutical doses comprising a container, filled with a composition including an active agent, a HFA propellant and a carrier.

WO 0 030 608 refers to pressurized metered dose inhaler containing a solution of an active ingredient in a HFA propellant, a co-solvent and optionally a low-volatility component **characterised in that** part or all of the internal surfaces of said inhalers consist of stainless steel, anodised aluminum or are lined with an inert organic coating.

WO 0 149 350 discloses a device for delivering metered aerosols comprising an active ingredient in solution in a propellant consisting of a HFA selected from HFA 134a, HFA227 or mixtures thereof, a co-solvent such as ethanol and optionally a low-volatility component, said device comprising a flat body with a seat for housing the can, an inhalation mouthpiece and an expansion chamber shaped to create a vortex flow of the aerosol particles expelled by the actuator.

WO 0 162 227 refers to a formulation for use in a metered dose aerosl inhaler, comprising an anticholinergic quaternary ammonium salt in solution in a mixture consisting of a hydrofluoroalkane propellant, a cosolvent and a low volatility component.

WO 0 166 089 relates to an alternative mode of administration for cannabis and its natural and synthetic derivatives, consisting in a formulation for sublingual delivery in aerosol or spray form.

### Disclosure of the invention

The aim of providing solution formulations in a HFA propellant for aerosol delivery of medicaments is to give a prompt systemically active dose of said medicament via the respiratory tract.

Hereinafter the term medicament is used to define any pharmaceutical active compound which could take advantage from a pulmonary delivery so as to produce a systemic therapeutic effect.

In order to provide therapeutically useful plasma levels, a therapeutic concentration of medicament and an efficient aerosol delivery should be achieved.

An important parameter for an efficient aerosol delivery to produce a systemic therapeutic effect is the particle size distribution in the aerosol cloud. When the formulation is in the form of suspension, the particle size of the cloud is dominated by the particle size of the suspended drug, defined by the milling/micronization process.

When the formulation is in the form of solution, the volumetric contribution of suspended drug particles is absent and much finer liquid droplets clouds, largely defined by the drug concentration in the solution, are generated.

The size of the particles provided by the pMDI, is normally expressed as mass median aerodynamic diameter (MMAD). The particle size of choice of aerosol medicaments for the treatment of bronchopulmonary diseases is usually of approximately 3 µm. The preferred diameter of the aerosol particles or droplets is comprised between 0.5 and 5 µm.

When the medicament is delivered to the lungs through an aerosol metered dose inhaler so as to produce a systemic effect, the particles should be small enough to be delivered to the lungs and to be absorbed into the bloodstream upon inhalation, i.e. of a size advantageously comprised between about 0.5 µm and 2.5 µm (MMAD of about 1-2 µm). Particles smaller than 0.5 µm are indeed not therapeutically useful as they are exhaled.

The aerosol solution formulations offer the advantage of being homogeneous with the active ingredient and with the excipients which are completely dissolved in the propellant vehicle or in the mixtures thereof with suitable co-solvents such as ethanol. Solution formulations also obviate physical stability problems associated with suspension formulations, thus assuring reproducible dosage.

Furthermore, when a systemic effect is required, as in the case of the invention, aerosol solution formulations offer the advantage that much finer clouds, largely defined by the drug concentration in the solution, are generated and the finer clouds give more extensive deposition in the lung periphery.

When a medicament is slightly soluble in HFA propellants such as HFA 134a and HFA 227 or in their mixture, the use of a solvent, generally ethanol is necessary.

When a medicament is very slightly soluble in the propellant large amounts of ethanol are required. A large amount of ethanol, in turn, increases, proportionally to its concentration, the size of the aerosol droplets leaving the actuator orifice. The larger size droplets extensively deposit into the oropharyngeal tract to the detriment of the drug dose fraction which penetrates into the lower airways (respirable fraction). A poorly respirable fraction is unlikely to give the medicament serum levels necessary to produce a therapeutic effect.

Moreover, an increased amount of ethanol in the formulation means also an increased amount of residual water. Whereas an amount of water up to 10% w/w, preferably comprised between 0.5 and 8% w/w and more preferably between 0.5 and 6% may be in some cases useful to improve the solubility of the medicament in the propellant/co-solvent system, in other cases the presence of water could enhance the degradation of the medicament and could be detrimental to the physical stability of the formulation giving rise to a nonhomogeneous system.

It would be advantageous to provide a formulation for pulmonary delivery to be used with pressurised metered dose inhalers, which is chemically and physically stable and capable of providing, on actuation, a suitable fine particle dose (FPD) and a fine respirable fraction (FPF) providing early therapeutic plasma levels of a medicament. The fine particle dose or respirable dose is the amount of active particles of size less than 4.7 µm and the fine particle fraction or respirable fraction is the ratio between the respirable dose and the dose delivered on actuation of the inhaler. The respirable fraction should be at least 30%, preferably more than 40%, even more preferably higher than 50% of the delivered dose.

It would also be highly advantageous to provide formulation whose delivered dose is highly reproducible after repeated administrations from the pMDI.

Since a high systemic exposure of the aerosol particles would, in this case, be of benefit, it would be even more advantageous to provide a formulation wherein the composition of the whole solvent system has been adjusted in order to allow the generation of aerosol particles which could then allow a deep lung penetration, at the same time minimizing the amount of very small particles (≤0.5 µm) which would be exhaled.

The invention provides a solution to said problems by means of solution formulations comprising a composition as claimed in claim 1. Said solutions are chemically stable for an adequate time and capable of providing, on actuation, a respirable fraction giving rise to onset-hastened therapeutic plasma levels of the medicament.

The preferred co-solvents is Ethanol.

The polarity may be quantified, and thus compared, in terms of a dielectric constant, or by using Maxwell's equation to relate dielectric constant to the square of the refractive index - the refractive index of materials being readily measurable or obtainable from the literature. Alternatively, the polarity of co-solvents may be measured using the Kauri-butanol value for estimation of solvent power. The protocol is described in ASTM Standard: Designation 1133-86.

The addition of a co-solvent with a higher polarity than ethanol allows reduction in the ethanol amount allowing the modulation of the particle size of the produced aerosol droplets.

Co-solvents with a higher polarity than ethanol can be preferably selected from lower alkyl (C₁-C₄) alcohol, polyols or polyalkylene glycols.

The preferred polyols include propylene glycol and glycerol and the preferred polyalkylene glycol is polyethylene glycol.

Among the co-solvents with a higher polarity than ethanol water is to be considered comprised. The amount of water, when present, is up to 10% w/w, preferably comprised between 0.5 and 8% w/w and more preferably between 0.5 and 6%.

Small amounts of ethanol and of a co-solvent are useful also when the medicament is fully soluble in the propellant.

It has been indeed found that, although the solvent is not needed to dissolve the medicament in the propellant, a small amount of ethanol (preferably comprised around 5-8% w/w, more preferably around 5% w/w), influencing the deposition characteristics, may make systemic delivery easier, since ethanol helps the reduction of the amount of very small particles (<0.5 µm) which would be exhaled due to a short residency time in the lung. Moreover, ethanol reduces the deposition of discharged material on the inhaler actuator orifice, so improving the dose reproducibility after repeated administrations by keeping *'clean'* the actuator orifice.

Due to this "cleaning" effect of ethanol generally the use of surface active agents or "surfactants" as valve lubricants is not necessary.

In certain cases, however, the formulation may optionally contain small amounts of additional components such as surfactants or other additives which are preservatives, buffers, antioxidants, radical quenchers, sweeteners and taste masking agents.

The preferred organic surfactant is selected from oleyl alcohol, sorbitan trioleate, sorbitan mono-oleate, sorbitan monolaurate, polyoxyethylene (20) sorbitan monolaurate, polyoxyethylene (20) sorbitan mono-oleate, natural lecithin, oleyl polyoxyethylene (2) ether, stearyl polyoxyethylene (2) ether, lauryl polyoxyethylene (4) ether, block copolymers of oxyethylene and oxypropylene, oleic acid, synthetic lecithin, diethylene glycol dioleate, tetrahydrofurfuryl oleate, ethyl oleate, isopropyl myristate, glyceryl mono-oleate, glyceryl monostearate, glyceryl monoricinoleate, cetyl alcohol, stearyl alcohol, cetyl pyridinium chloride, olive oil, glyceryl monolaurate, corn oil, cotton seed oil or sunflower seed oil.

According to a further aspect, the invention provides a method of filling an aerosol inhaler with a composition of the invention, the method comprising:
(a) weighing the required quantity of active ingredient into the can or vial;
(b) adding the appropriate volume of ethanol and of an additional co-solvent, if required;
(c) crimping with valves and gassing;
(d) adding a propellant containing a hydrofluoroalkane (HFA).

The high efficiency cloud generation allows to prepare formulations containing a medicament with a reduced nominal dose and a larger percentage of clinically useful medicament deposition with respect to the reference composition (FPF of at least 30%, preferably higher than 40%, even more preferably more higher than 50% of the delivered dose) and with defined particle size targeting specific areas of the lungs.

Said medicaments can optionally be used in the form of their esters, isomers, enantiomers o racemates and, in the case of acids or bases, as such or in the form of their pharmaceutically acceptable salts.

Advantageously, the concentration of the active ingredient is at least 0.01 % w/v, preferably at least 0.05% w/v, more preferably between 0.1 % w/v and 1.0% w/v, even more preferably at least 1.0% w/v.

It is preferable that the formulation is suitable for delivering a therapeutic amount of the active ingredient in one or two actuations. Advantageously the formulation will be suitable for delivering a therapeutic dose of at least 25 µg/dose, preferably between 50 and 500 µg/dose. By "therapeutic dose" it is meant the amount of active ingredient delivered by a single actuation of the inhaler able to produce a pharmacodynamic effect.

The formulations of the invention could be filled into cans suitable for delivering pharmaceutical aerosol formulations. Certain medicaments are subject to enhanced chemical degradation when stored in contact with the standard metal container usually made of aluminium. In these cases the formulations will be filled preferably into cans having part or all of the internal surfaces made of anodised aluminium, stainless steel or lined with an inert organic coating. Examples of preferred coatings are epoxy-phenol resins, perfluoroalkoxyalkane, perfluoroalkoxy alkylene, perfluoroalkylenes such as polytetrafluoro-ethylene, fluorinated-ethylene-propylene, polyether sulfone and a copolymer fluorinated-ethylene-propylene polyether sulfone. Other suitable coatings could be polyamide, polyimide, polyamideimide, polyphenylene sulfide or their combinations.

To further improve the stability, cans having a rolled-in rim and preferably a part or full rollover rim are used.

The formulation is actuated by a metering valve capable of delivering a volume of between 25 µl and 100 µl.

The choice of the metering valve and type of gasket will be made according the knowledge of the person skilled in the art. The gasket may comprise any suitable elastomeric material such as low density polyethylene, EPDM, chloroprene and TPE.

Suitable valves are commercially available from manufacturers well known in the aerosol industry, for example from Valois, France, Bespak plc, UK and 3M, Neotechnic Ltd, UK.

For reasons of chemical stability of the medicament in solution, it is preferred in some cases that the internal surfaces of metal valve components in contact with the formulation are coated with an inert material.

The currently used valve actuators with orifice diameter from 0.20 to 0.50 mm (and in particular 0.22, 0.33, 0.42 and 0.45 mm) can be generally used with the aerosol formulations of the invention. When large amounts of ethanol are required to dissolve the medicament, so as to obtain aerosol clouds with an optimal respirable fraction, valve actuators provided with orifice diameters comprised between 0.10-0.20 mm (and in particular 0.12, 0.14, 0.16, 0.18 mm) are advantageously used.

These kinds of orifices can be prepared according to the EP application n° 01 130521.6 in the Applicant's name.

In some cases, in order to stabilise the medicament in solution, it would be necessary to provide aerosol solutions with a specific apparent pH, which can be determined by the skilled in the art according to WO 01/894080.

The hydrofluorocarbon propellant is selected from the group of HFA 134a, HFA 227 and mixtures thereof.

The co-solvent may include one or more solvents and in this case their ratio is a critical factor for an efficient aerosolization. The selection of said ratios may be anyhow made by the skilled in the art on the basis of the chemico-physical characteristics of the considered medicament.

The preferred co-solvents are usually alcohols such as ethanol, propanol, propylene glycol, polyethylene glycol, glycerol and their mixture in a total amount up to 30% w/w, preferably up to 25% w/w, more preferably up to 20% w/w.

Another useful co-solvent in some kinds of formulations is water.

Advantageously, the droplets size is between about 0.5 µm and 2.5 µm, corresponding to a MMAD of about 1-2 µm.

### Preparation of HFA Solution pMDIs

The assembly of the pMDI cans was carried out using hand operated crimping and filling equipment. Formulations were prepared by accurately weighing the required quantity of drug into the can or vial. The appropriate volume of ethanol and the other co-solvent if required in the formulation, was then added. The valve was crimped onto the vial/can and the assembled vial/can was ultra-sonicated for approximately 10 minutes. The HFA propellant was filled through the valve and the pMDI was ultra-sonicated for a further 10 minutes. In the case of formulations that contained drug and propellant only the pMDI was ultra-sonicated once, after the propellant had been added. Final compositions were calculated as percentage w/v for the active ingredient and as percentage w/w for the co-solvents.

### Solubility Studies

All solubility investigations were conducted in plastic coated glass pMDI vials fitted with continuous spray valves. Once produced the medicament-HFA solution pMDIs were stored in refrigerator at 4°C (± 0.1°C). The pMDI vials were removed periodically and the vials assessed visually with the aid of a polarized light unit for crystal growth.

### Cascade Impaction Studies

All impaction studies were conducted with formulations contained in cut edge anodised aluminium cans fitted with 50 µl or 100 µl valves. The studies were carried out using an Andersen Cascade Impactor (ACI) fitted with a USP XXII metal throat entry port.

The ACI was operated at a flow rate of 28.3 ± 2 1 min ⁻¹. The HFA solution formulations were discharged into the ACI through actuators having an orifice diameter from 0.14 to 0.45 mm. Deposition of the drug on each ACI plate was determined by high pressure liquid chromatography (HPLC).

MMAD values and corresponding geometric standard deviation (GSD) were calculated from plots of the cumulative percentage undersize of drug collected on each ACI plate (probit scale), against the upper cut off diameter for each respective ACI plate (log10 scale).

The following parameters were determined: the metered dose, which is the sum of the dose delivered through the Andersen apparatus plus the active ingredient residue deposited on the device actuator; the cumulative amount of active particles deposited on the various ACI stages; the amount on the actuator; the amount in the adaptor and in the throat (adp/throat); the fine particle dose or respirable dose (FPD) which is the amount of particles deposited on stages 3 to filter of the ACI and corresponds to the amount of particles of size less than 4.7 µm; the fine particle fraction or respirable fraction which is the ratio between the respirable dose and the dose delivered ex-actuator.

Examples of formulations according to the invention comprise:
- apomorphine esters in a HFA propellant selected from HFA 134a, HFA 227 and their mixtures and a co-solvent selected from an alcohol, a polyol and their mixtures. In a particular embodiment the formulation comprises up to 1% w/v diisobutyryl apomorphine, up to 5% w/w ethanol, from 0 to 0.1% w/w glycerol and HFA 134a,.

### Example 1

### Solubility studies of diisobutyryl apomorphine, aerosol delivery characteristics and stability of its corresponding pMDI formulations Solubility studies

The solubility of diisobutyryl apomorphine was investigated by producing pMDI formulations at various percentages of ethanol in HFA 134a or in HFA 227.

The results showed that formulations containing up to 1% w/v diisobutyryl apomorphine are soluble in HFA 134a or HFA 227.

### Aerosol delivery characteristics studies

0.5% and 1% w/v (250 µg or 500 µg/50 µl respectively) diisobutyryl apomorphine HFA 134a solution formulations containing 5% w/w ethanol and 0.1% w/w glycerol were produced. The cans were provided with actuators with an orifice diameter of 0.22 mm.

Two ACI deposition determinations were performed with each formulation. Twenty shots were discharged into the ACI.

The diisobutyryl apomorphine formulations prepared according to the invention presented a MMAD of about 2.0 µm, a fine particle fraction (FPF) of at least 70-75%, whereas the amount of active particles of sizes included in the range from 0.43 to 3.3 µm was of at least of 60%.

### Stability study

A stability study on a formulation prepared according to the Example 1 was initiated storing coated aluminum cans upright and inverted at 25°C.

The recovery of diisobutyryl apomorphine was determined by HPLC.

At six months evaluation the recovery of the active ingredient was excellent and minimal degradation occurred. There was no significant difference between those cans stored upright and inverted.

### Example 2 ( Reference example )

### Solubility studies of leuprolide acetate, aerosol delivery characteristics and stability of its corresponding pMDI formulations Solubility studies

The solubility of leuprolide acetate was investigated by producing pMDI formulations at various percentages of ethanol and water in HFA 134a or in HFA 227.

The results showed that formulations containing up to 0.26% w/v leuprolide acetate are soluble in ethanol, water, HFA 134a systems.

When water was added a significant increase of leuprolide acetate solubility within ethanol/HFA 134a systems was obtained.

### Aerosol delivery characteristics studies

0.04% w/v (40 µg/100 µl) leuprolide acetate HFA 134a solution formulation containing 15% w/w ethanol and 2% w/w water was produced. The cans were provided with actuators with an orifice diameter of 0.14 mm.

Two ACI deposition determinations were performed with the formulation. Ten shots were discharged into the ACI.

The leuprolide acetate formulation prepared according to the invention presented a MMAD of about 1.0 µm, a fine particle fraction (FPF) of at least 72%, whereas the amount of active particles of sizes included in the range from 0.43 to 3.3 µm was of at least of 61 %.

Another HFA 134a solution formulation containing 0.08% w/v (80 µg/100 µl) leuprolide acetate, 18% w/w ethanol and 3% w/w water was produced. The cans were provided with actuators with an orifice diameter of 0.14 mm.

Two ACI deposition determinations were performed with the formulation. Ten shots were discharged into the ACI.

The leuprolide acetate formulation prepared according to the invention presented a MMAD of about 1.3 µm, a fine particle fraction (FPF) of at least 59%, whereas the amount of active particles of sizes included in the range from 0.43 to 3.3 µm was of at least of 52%.

### Stability Studies

Stability studies on leuprolide 100 µg/50 µl HFA 134a pMDIs containing 30% w/w ethanol and 5% w/w water was initiated storing coated aluminum cans upright and inverted at 25°C.

The leuprolide acetate content was determined by HPLC.

Excellent stability was observed over the six-month stability study.

## Claims

1. An aerosol inhaler filled with a pharmaceutical formulation for aerosol administration of diisobutyryl apomorphine, being dissolved in a solution of a hydrofluoroalkane propellant, including diisobutyryl apomorphine, one or more hydrofluoroalkanes selected from HFA 134a and HFA 227, ethanol in an amount up to 5% w/w and glycerol in an amount from 0 to 0.1 % w/w.

2. An aerosol inhaler according to claim 1, wherein the fine particle fraction of medicament delivered on actuation of the inhaler is of at least 70-75% of the delivered dose.

3. An aerosol inhaler according to claim 1, or 2, wherein the mass median aerodynamic diameter is of 2.0 µm.

4. An aerosol inhaler according to any one of claims 1 to 3, wherein the particle size is of at least 60% from 0.43 to 3.3 µm.

5. An aerosol inhaler according to any one of claims 1 to 4, wherein the actuator has an orifice diameter of 0.22 mm.

## Patentansprüche

1. Aerosol-Inhalator, gefüllt mit einer pharmazeutischen Formulierung zur Aerosolverabreichung von Diisobutyrylapomorphin, welches in einer Lösung eines Hydrofluoralkan-Treibmittels gelöst ist, umfassend Diisobutyrylapomorphin, ein oder mehr Hydrofluoralkane, ausgewählt aus HFA 134a und HFA 227, Ethanol in einer Menge bis zu 5% G/G und Glycerin in einer Menge von 0 bis 0,1% G/G.

2. Aerosol-Inhalator nach Anspruch 1, wobei die Fraktion feiner Partikel an Medikament, die bei Betätigung des Inhalators abgegeben wird, wenigstens 70-75% der abgegebenen Dosis ist.

3. Aerosol-Inhalator nach Anspruch 1 oder 2, wobei der massenmittlere aerodynamische Durchmesser 2,0 µm ist.

4. Aerosol-Inhalator nach einem der Ansprüche 1 bis 3, wobei die Partikelgröße von wenigstens 60% 0,43 bis 3,3 µm ist.

5. Aerosol-Inhalator nach einem der Ansprüche 1 bis 4, wobei das Betätigungselement einen Öffnungsdurchmesser von 0,22 µm hat.

## Revendications

1. Inhalateur aérosol rempli d'une formulation pharmaceutique pour administration par aérosol de diisobutyrylapomorphine, la diisobutyrylapomorphine étant dissoute dans une solution de propulseur hydrofluoroalcane, incluant de la diisobutyrylapomorphine, un ou plusieurs hydrofluoroaloanes choisis parmi HFA 134a et HFA 227, de l'éthanol dans une quantité allant jusqu'à 5 % p/p et du glycérol dans une quantité de 0 à 0,1 % p/p.

2. Inhalateur aérosol selon la revendication 1, dans lequel la fraction de particules fines de médicament délivré lors d'un actionnement de l'inhalateur est d'au moins 70 à 75 % de la dose délivrée.

3. Inhalateur aérosol selon la revendication 1 ou 2, dans lequel le diamètre médian aérodynamique en masse est de 2,0 µm.

4. Inhalateur aérosol selon l'une quelconque des revendications 1 à 3, dans lequel au moins 60% des particules ont une taille de 0,43 à 3,3 µm.

5. Inhalateur aérosol selon l'une quelconque des revendications 1 à 4, dans lequel l'actionneur possède un diamètre d'orifice de 0,22 mm.
